(19) Europäisches Patentamt
European Patent Office
Office européen des brevets

(11) Numéro de publication: **0 448 550 B1**

(12) # FASCICULE DE BREVET EUROPEEN

(45) Date de publication de fascicule du brevet: **10.08.94**

(51) Int. Cl.5: **C07H 19/073**, A61K 31/70

(21) Numéro de dépôt: **89903177.7**

(22) Date de dépôt: **23.02.89**

(86) Numéro de dépôt internationale :
**PCT/FR89/00072**

(87) Numéro de publication internationale :
**WO 89/08115 (08.09.89 89/21)**

(54) **NOUVEAUX DERIVES DE LA DEOXY-2'-URIDINE SUBSTITUEE EN POSITION 5, 3' ou 5' PAR DES GROUPEMENTS ALPHA-AMINO ACYLES, PROCEDE POUR LEUR OBTENTION ET MEDICAMENTS LES CONTENANT.**

(30) Priorité: **24.02.88 FR 8802255**
**28.06.88 FR 8808684**

(43) Date de publication de la demande:
**02.10.91 Bulletin 91/40**

(45) Mention de la délivrance du brevet:
**10.08.94 Bulletin 94/32**

(84) Etats contractants désignés:
**AT BE CH DE FR GB IT LI LU NL SE**

(56) Documents cités:
**US-A- 3 853 845**
**US-A- 4 128 639**

**Approches to antiviral agents pp.60.69 (1985).**

(73) Titulaire: **INSTITUT DE RECHERCHES CHIMIOUES ET BIOLOGIOUES APPLIOUEES (I.R.C.E.B.A.) Société à responsabilité limitée dite:**
**62, Grande-Rue**
**F-78490 Vicq (FR)**

(72) Inventeur: **MORINIERE, Jean-Luc**
**43, avenue Arnold-Netter**
**F-75012 Paris (FR)**
Inventeur: **FAULOUES, Michelle**
**15, rue de Chanzy**
**F-92400 Courbevoie (FR)**
Inventeur: **ROUSSEAU, Claude**
**30, Grande-Rue**
**F-78190 Orgerus (FR)**
Inventeur: **DANREE, Bernard**
**53, rue des Grands-Champs**
**F-78300 Poissy (FR)**
Inventeur: **MAROUER, Claude**
**112, rue des Landes**
**F-78400 Chatou (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

Journal of the American Chemical Society, Volume 93, Nr. 6, 24 March 1971, (Columbus, Ohio, US), M.J. Robins et al.: "Nucleoside peptides. I. The synthesis of 5'-deoxy-5'-N-aminoacyl peptide derivatives of guanosine, adenosine, and 2'-deoxyadenosine and their effect on cell-free protein synthis" pages 1474-1480 see the whole document

Journal of Medicinal Chemistry, Volume 22, Nr. 6, 1979, American Society, (Columbus, Ohio, US), A. Hempton et al.: "Design of species- or isozyme-specific enzyme inhibitors. 1. Effect of thymidine substituents on affinity for the thymidine site of hamster cytoplasmic thymidine kinase", pages 621-631 see page 623

Journal of Medical Chemistry, Volume 29, Nr. 6, 1986, American Chemical Society, (Columbus, Ohio, US), R.D. Elliott et al.: "Reactive 5'-substituted thymidine derivatives as potential inhibitors of nucelotide biosynthesis", pages 1052-1056 see page 1053

Journal of Medicinal Chemistry, Volume 30, Nr. 5, 1987, American Chemical Society, (Columbus, Ohio, US), R.D. Elliot, et al.: "Reactive 5'- substituted 2', 5'-dideoxyuridine derivatives as potential inhibitors of nucleotide biosynthesis", pages 927-930, see page 928

Journal of Medicinal Chemistry, Volume 21, Nr. 1, 1978, American Chemical Society, (Columbus, Ohio, US), T.-S. Lin et al.: "Synthesis and biological activity of several amino analogues of thymidine", pages 109-112 see page 110, compond 8 cited in the application.

Journal of Medicinal Chemistry, Volume 22, Nr. 12, 1979, American Chemical Society, (Columbus, Ohio, US), A. Hampton et al: "Design of species- or isozyme-specific enzyme inhibitors. 2. Differences between a bacterial and a mammalian thymidine kinase in the effect of thymidine substituents on affinity for the thymidine site", pages 1524-1528 see compond 6b

Inventeur: **SAUR, Patrick**
**15, rue des Bleuets**
**F-94550 Chevilly-la-Rue (FR)**
Inventeur: **LEMOINE, Jean**
**5, rue Yvan-Tourqueniev**
**F-78380 Bougival (FR)**
Inventeur: **LACOLLE, Jean-Yves**
**Résidence du Parc**
**F-78860 Saint-Nom-la-Bretèche (FR)**

(74) Mandataire: **Portal, Gérard et al**
**Cabinet Beau de Loménie**
**158, rue de l'Université**
**F-75340 Paris Cédex 07 (FR)**

## Description

La présente invention a pour objet de nouveaux dérivés de la déoxy-2'-uridine substituée en position 5, 3' ou 5' par des groupements α-amino acylés, un procédé pour les préparer et des compositions pharmaceutiques les contenant.

On connaît déjà certains dérivés de la déoxy-2'-uridine.

C'est ainsi qu'ont été décrits des dérivés de la déoxy-2'-uridine substituée en position 5, et répondant à la formule chimique générale suivante :

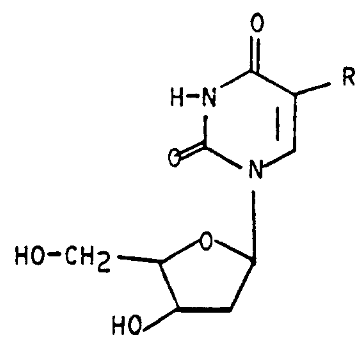

dans laquelle

R est un radical alkyle ou alcényle ayant de 1 à 4 atomes de carbone, un radical aryle ou un halogène, lesdits radicaux alkyle et aryle pouvant comporter au moins un substituant halogène.

On connaît également, notamment par le brevet américain 4 093 716, l'amino-5'-didéoxy-2',5'-méthyl-5-uridine de formule :

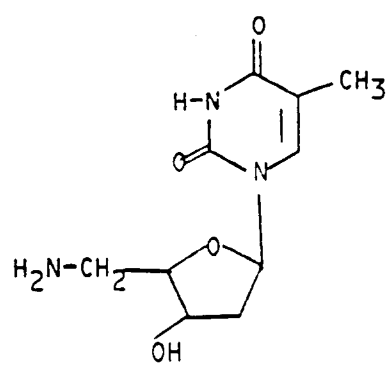

qui est présenté comme inhibiteur potentiel du virus de l'herpès. La préparation de ce composé est décrite dans un article d'Horwitz et al., dans la revue "J. Am. Chem. Soc." 27 3045, (1962).

On connaît également, notamment par une publication de LIN et PRUSOFF (J. Med. Chem. 21 109 (1978)), l'amino-3'-didéoxy-2',3'-méthyl-5-uridine de formule :

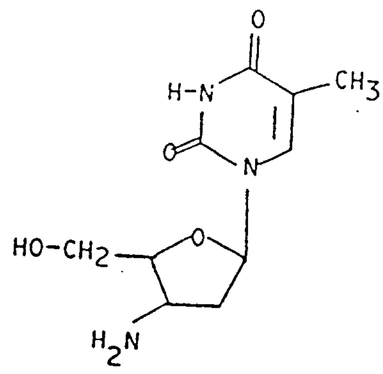

On connaît enfin, notamment par une publication de BELTZ et VISSER (J. Biol. Chem., 226, 1035, (1957)), l'amino-5-déoxy-2'-uridine de formule :

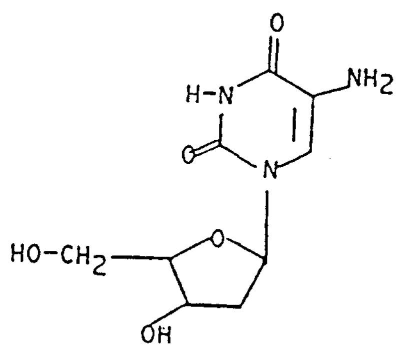

Le document J. Med. Chem. 1979, vol 22, No 6, pages 621 à 631 décrit généralement un procédé pour l'obtention de 5-[[ω-(Iodoacétamido)acyl]amino]-2'-déoxyuridines, utilisant, en tant qu'intermédiaires de synthèse des composés de formule :

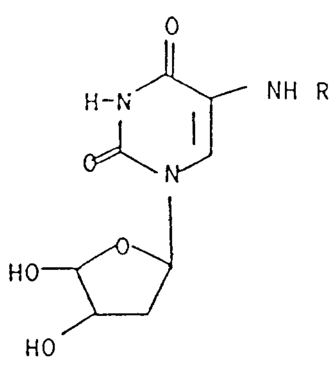

dans laquelle R représente le reste d'un ω-amino acide.

Ces composés intermédiaires ne sont pas présentés comme susceptibles d'application thérapeutique.

La présente invention concerne une nouvelle classe de dérivés de la déoxy-2'-uridine substituée en position 5, 3' ou 5' par des groupements α-amino acylés, répondant à la formule générale suivante :

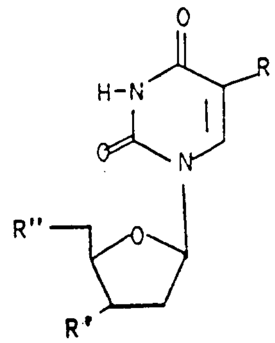

(I)

dans laquelle

. R est choisi parmi un radical alkyle ou alcényle ayant de 1 à 4 atomes de carbone, un radical aryle ou un halogène, lesdits radicaux alkyle, alcényle et aryle pouvant comporter au moins un substituant halogène ; et un radical de formule -NH-$R_1$, dans lequel $R_1$ représente un reste d'acide aminé ou de peptide comportant de 2 à 6 acides aminés ;

. R' et R'' étant choisis parmi un radical hydroxy et un radical de formule -NH-$R_1$, dans lequel $R_1$ a la même signification que ci-dessus ;

à la condition que R' et R'' ne soient pas simultanément -NH-$R_1$ et que, lorsque R est -NH-$R_1$, R' et R'' soient simultanément un groupe hydroxy; et leurs sels acceptables en pharmacie.

L'invention comprend également dans son cadre tous les isomères optiques possibles des composés de formule (I) ainsi que leurs mélanges.

Dans la formule générale (I), un atome d'halogène est de préférence un atome de chlore ou de brome.

Les groupes alkyles, alcényles peuvent être des groupes à chaîne droite ou ramifiée.

Un groupe alkyle ayant de 1 à 4 atomes de carbone est par exemple un groupe méthyle, éthyle, propyle, isopropyle, butyle, sec.-butyle ou tert.-butyle, de préférence méthyle ou éthyle.

Un groupe alcényle ayant de 1 à 4 atomes de carbone est par exemple un groupe vinyle, propényle ou butényle, de préférence vinyle.

Les sels acceptables en pharmacie des composés de formule (I) comprennent ceux formés avec un acide minéral, par exemple l'acide chlorhydrique ou l'acide sulfurique ou avec un acide organique, par exemple l'acide citrique, tartrique, malique, maléique ou fumarique.

Il a été découvert, de façon tout à fait surprenante, que ces nouveaux dérivés possèdent la propriété pharmacologique intéressante d'être des inhibiteurs de la thymidine kinase foetale présente dans les tissus cancéreux humains et sont ainsi des inhibiteurs de la synthèse de l'ADN des cellules cancéreuses en voie de prolifération.

Selon une caractéristique particulière, l'invention concerne la nouvelle classe de dérivés de la déoxy-2'-uridine substituée en position 5', c'est-à-dire les composés de formule générale (I), dans laquelle R' est un groupe hydroxy et R'' est un groupe -NH-$R_1$.

Selon une autre caractéristique particulière, l'invention concerne la nouvelle classe de dérivés de la déoxy-2'-uridine substituée en position 3', c'est-à-dire les composés de formule générale (I), dans laquelle R' est un groupe -NH-$R_1$ et R'' est un groupe hydroxy.

Selon encore une autre caractéristique particulière, l'invention concerne la nouvelle classe de dérivés de la déoxy-2'-uridine substituée en position 5, c'est-à-dire les composés de formule générale (I), dans laquelle R est un groupe -NH-$R_1$, R' et R'' étant simultanément des groupes hydroxy.

Les nouveaux composés de formule (I) selon l'invention se présentent, suivant la structure de l'acide aminé ou du peptide précité, sous la forme D ou L, ou encore DL.

Le procédé général pour la préparation des composés selon l'invention de formule générale (I) telle que définie précédemment comporte la réaction, par voie enzymatique ou par voie chimique, d'un dérivé aminé répondant à la formule générale :

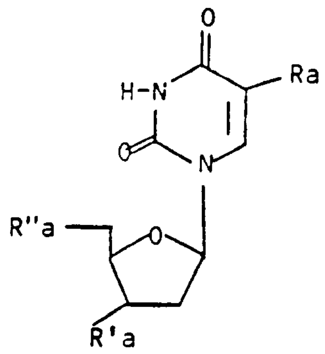

(Ia)

dans laquelle

$R_a$ est choisi parmi un radical alkyle ou alcényle ayant de 1 à 4 atomes de carbone, un radical aryle ou un halogène, lesdits radicaux alkyle, alcényle et aryle pouvant comporter au moins un substituant halogène, et un radical $-NH_2$ ;

$R'_a$ et $R''_a$ étant choisis parmi un radical hydroxy et un radical $-NH_2$,

à la condition que $R'_a$ et $R''_a$ ne soient pas simultanément $-NH_2$ et que, lorsque $R_a$ est $-NH_2$, $R'_a$ et $R''_a$ soient simultanément un groupe hydroxy ;

avec un acide aminé ou un peptide comportant de 2 à 6 acides aminés, et, si on le désire, la conversion d'un composé ainsi obtenu en un sel acceptable en pharmacie.

Les composés de forme L sont préparés de préférence par réaction enzymatique des dérivés aminés de formule (Ia) avec un acide aminé ou un peptide de forme L ou DL, en présence d'une enzyme choisie parmi la papaïne et la chymopapaïne, la réaction étant effectuée à une température d'environ 10 à environ 70 °C en milieu acide. Dans ces conditions, il y a réaction entre la fonction amine du dérivé aminé en 5, en 3' ou en 5' et la fonction acide libre de l'acide aminé ou du peptide, étant entendu que les fonctions amines dudit acide aminé ou peptide sont préalablement bloquées à l'aide de réactifs connus tels que le benzyloxycarbonyle, le paraméthoxybenzyloxycarbonyle, le t-butoxycarbonyle ou le fluorénométhyloxycarbonyle.

Les composés de forme L, D ou DL peuvent également être préparés en utilisant un procédé purement chimique comportant éventuellement la séparation d'un mélange d'isomères des composés de formule (I) en les isomères isolés.

De façon surprenante, les composés de formule (I) selon l'invention se sont révélés actifs en inhibant la synthèse de l'ADN des cellules cancéreuses et de certaines particules virales.

Ces composés sont donc notamment utiles dans le traitement des cancers hormonodépendants et d'infections d'origine virale.

On emploie en général la voie orale pour tous les états nécessitant de tels composés. A cet effet, les composés de l'invention peuvent être administrés à des doses comprises par exemple entre environ 30 $mg/m^2$ et environ 100 $mg/m^2$ par jour dans le traitement des cancers, et par exemple entre environ 50 mg/kg et environ 250 mg/kg par jour, dans le traitement d'infections d'origine virale. Bien entendu, ces posologies peuvent être ajustées pour assurer la réponse thérapeutique optimale.

Ainsi, selon un dernier aspect, la présente invention concerne des compositions pharmaceutiques, notamment à activité d'inhibiteur de la synthèse de l'ADN des cellules cancéreuses en voie de prolifération, caractérisées en ce qu'elles contiennent, à titre d'ingrédient actif, au moins l'un des composés définis précédemment de formule générale I, en association avec un véhicule ou support non toxique pharmaceutiquement acceptable.

Les compositions pharmaceutiques de l'invention sont généralement préparées selon des procédés classiques et sont administrées sous une forme pharmaceutique appropriée, par exemple une forme orale solide peut contenir, avec le composé actif des diluants, par exemple le lactose, la cellulose ; des lubrifiants, par exemple l'acide stéarique ou des polyéthylèneglycols ; des agents liants tels que des amidons, la méthylcellulose ; des colorants ; des édulcorants ; des agents mouillants comme par exemple les polysorbates ; et, en général, les substances non toxiques et pharmacologiquement inertes utilisées dans les compositions pharmaceutiques.

L'invention sera illustrée plus en détail par les exemples non-limitatifs suivants :

Exemple 1 : Voie enzymatique

Synthèse de N-(L-alanyl)-amino-5'-didéoxy-2',5'-méthyl-5-uridine

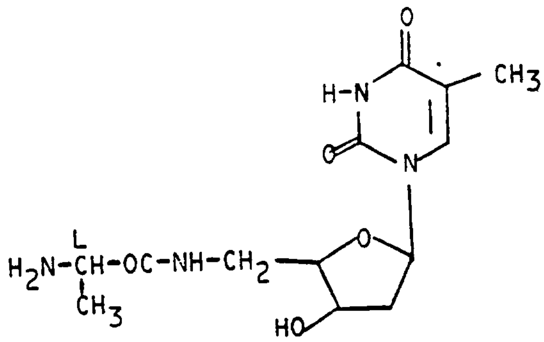

Etape A : Synthèse de N-[(N-benzyloxycarbonyl)-L-alanyl]-amino-5'-didéoxy-2',5'-méthyl-5-uridine

Dans un tricol de 100 ml équipé d'une agitation magnétique, d'un thermomètre et d'un réfrigérant, on introduit successivement :

- 16 ml de soude normale
- 3,57 g de (N-benzyloxycarbonyl)-L-alanine (0,016 mole)
- 3,86 g d'amino-5'-didéoxy-2',5'-methyl-5-uridine (0,016 mole)
- 188 mg de chlorhydrate de L-cystéine dissous dans 2,4 ml d'eau distillée
- 20 ml de solution tampon citrique pH : 5

Le milieu réactionnel dont le pH est 8,98 est chauffé à 50 °C ; à partir de 30 °C, le mélange est limpide.

A 50 °C, on additionne une solution de 3,6 g de papaïne (2,9 UI/mg) dans 8 ml d'eau. Le pH évolue lentement vers 8,41.

Par additon d'acide citrique en poudre (m = 1,2 g) on amène le pH à 4,6 et laisse sous vive agitation pendant 24 h.

La réaction terminée, on refroidit le milieu réactionnel, filtre et lave le précipité à l'eau.

Après séchage à 60 °C sous vide, on obtient 4,3 g de N-[(N-benzyloxycarbonyl)-L-alanyl]-amino-5'-didéoxy-2',5'-méthyl-5-uridine.

La purification est effectuée par cristallisation dans 50 ml de méthanol.

m = 3,9 g     Rdt = 55 %     Pureté : 95 %

Etape B :

Ce composé est mis en solution dans le méthanol et hydrogéné en présence de 400 mg de charbon palladié à 10 %.

Après filtration du catalyseur et concentration du solvant, on reprend le résidu par l'éther éthylique et filtre le précipité obtenu.

On obtient après séchage 7,9 mmoles de N-(L-alanyl)-amino-5'-didéoxy-2',5'-méthyl-5-uridine (Rdt : 90 %). La recristallisation dans 20 ml de méthanol du produit précédent conduit à 7,11 mmoles du composé désiré optiquement pur. m = 2,21 g Rdt global : 44 % ; F = 190-193 °C (Thermovar) ; $[\alpha]_D^{20}$ = +28 °5 (C = 0,5 MeOH) ; IR (KBR) : 1670 $cm^{-1}$ (C = O) ; 1275 $cm^{-1}$, 1580 $cm^{-1}$, 3250 $cm^{-1}$ (N - H). RMN (200 MHz, $CD_3OD$) : 1,30 (3H, d, Me ala) ; 1,90 (3H, s, Me) ; 2,22 à 2,28 (2H, m, C-2'-H) ; 3,47 à 3,51 (3H, m, C-5'-H, C-ala-H) ;

3,90 à 3,92 (1H, m, C-4'-H) ; 4,25 à 4,27 (1H, m, C-3'-H) ; 6,18 (1H, t, C-1'-H) ; 7,49 (1H, s, C-6-H).

Exemple 2 : Voie chimique

Synthèse de N-(D-alanyl)-amino-5'-didéoxy-2',5'-méthyl-5-uridine

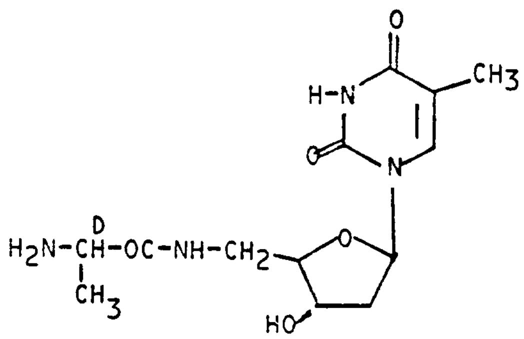

Etape A : Synthèse de N-[(N-benzyloxycarbonyl)-D-alanyl]-amino 5'-didéoxy-2',5'-méthyl-5-uridine.

Dans un tricol de 100 ml équipé d'une agitation magnétique, d'un thermomètre et d'une garde de chlorure de calcium, on introduit :

- 20 ml de dichlorométhane sec (tamis 4 Å)
- 893 mg de (N-benzyloxycarbonyl)-D-alanine (4 mmoles)
- 0,6 ml de triéthylamine (4,3 mmoles)

On refroidit la solution obtenue à -5 °C et ajoute 0,4 ml de chloroformiate d'éthyle (4,2 mmoles).

Après 90 minutes d'agitation, on additionne 964 mg d'amino-5'-didéoxy-2',5'-méthyl-5-uridine et laisse 60 minutes à -5 °C puis une nuit à température ambiante.

On filtre le précipité formé et le lave par 15 ml d'eau permutée. Après séchage à l'étuve sous vide à 60 °C, on obtient 1,33 g de N-[(N-benzyloxycarbonyl)-D-alanyl]-amino-5'-didéoxy-2', 5'-méthyl-5-uridine. Une cristallisation dans 50 ml de méthanol conduit à 1,2 g d'un composé de pureté satisfaisante (Rdt : 67 %).

Etape B :

L'hydrogénolyse et une purification analogues à celles décrites dans l'exemple 1, conduisent au N-(D-alanyl)-amino-5'-didéoxy-2',5'-méthyl-5-uridine optiquement pur [m = 715 mg ; 2,3 mmoles ; Rdt 57 %] F = 180-184 °C (Thermovar) $\alpha_D^{20}$ = + 22,6 ° (C = 1, MeOH) IR (KBr) : 1670 $cm^{-1}$ (C = O) ; 1275 $cm^{-1}$, 1580 $cm^{-1}$, 3250 $cm^{-1}$ (N-H) ; C.C.M. : (MeOH, $H_2O$ : 80/20) rf = 0,21 silice 60 $F_{254}$.

RMN (200 MHz, $D_2O$) : 1,23 - 1,30 (3H, d, Me ala) ;
1,90 (3H, s ; Me) ; 2,35 à 2,41 (2H, m, C-2'-H) ; 3,52 à 3,57 (3H, m C- 5'H, C ala - H) ; 4,02 à 4,04 (I H, m, C-4'-H) ; 4,37 à 4,40 (1H, m, C-3'H) ; 6,25 (1H, t, C-1'-H) ; 7,48 (11 H, s, C-6-H)

Exemple 3

Synthèse de N-(D-alanyl)amino-3'-didéoxy-2',3'-méthyl-5-uridine

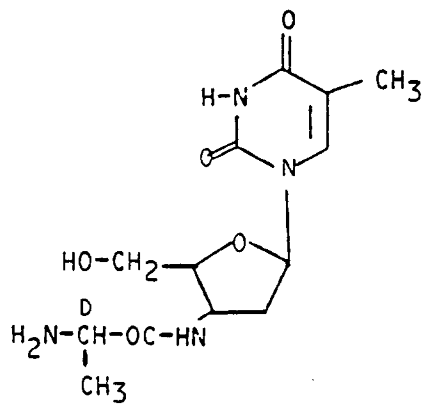

Le protocole opératoire est identique à celui décrit dans l'exemple 2.

Etape A : Synthèse de N-[(N-benzyloxycarbonyl)-D-alanyl]-amino-3'-didéoxy-2'-3'-méthyl-5-uridine (1)

Les produits de départ sont les suivants :

- 20 ml de dichlorométhane sec (tamis 4 Å)
- 925 mg de (N-benzyloxycarbonyl)-D-alanine (4,15 mmoles)
- 0,62 ml de triéthylamine (4,4 mmoles)

On obtient ainsi 0,95 g du composé (1) avec une pureté satisfaisante (Rdt : 64 %).
F = 124-128 °C (thermovar) : $\alpha^{20}_{D}$ = + 25° (c = 0,1 , MeOH)
RMN (200 MHz, CD3 OD) : 1,2 à 1,3 (3H, d, Me, ala) ; 1,7 (3H, s, Me) ; 2,29 à 2,32 (2H, m, C-2'-H) ; 3,7 à 3,9 (3H, m, C-5'-H, C4'-H) 4 à 4,1 (1H, d, C-ala H) ; 6,2 (1H, t, C-1'-H) ; 7,3 à 7,4 (5H, m) ; 7,9 (1H, s, C-6-H)

Etape B :

Ce composé (1) est mis en solution dans le méthanol et hydrogéné en présence de 200 mg de charbon palladié à 10 %.

Après filtration du catalyseur et concentration du solvant, on reprend le résidu par l'éther éthylique et filtre le précipité obtenu.

On obtient après séchage 502 mg de N-(D-alanyl)-amino-3'-didéoxy-2'-3'-méthyl-5-uridine (Rdt 90 %) F = 182-184 °C
$\alpha^{20}_{D}$ = +22,5° (C = 1, $H_2O$)
ccm :(MeOH, $H_2O$ : 80/20) rf = 0,26 Silice 60 $F_{254}$
RMN (200 MHz, $D_2O$) : 1,2 à 1,3 (3H, d, Me-ala) ; 1,9 (3H, s, Me) ; 2,4 (2H,m, C-2'-H) ; 3,7 (1 H, m, C-ala) ; 3,8 à 3,9 (2H, m, C-5'-H) ; 4 (1H, m, C-4'-H) ; 4,4 à 4,6 (1H, m, C-3'-H) ; 6,2 à 6,3 (1H, t, C-1'-H) ; 7,7 (1H, s, C-6-H)

Exemple 4 : Voie chimique

Synthèse de N-(L-alanyl)-amino-3'-didéoxy-2',3'-méthyl-5-uridine

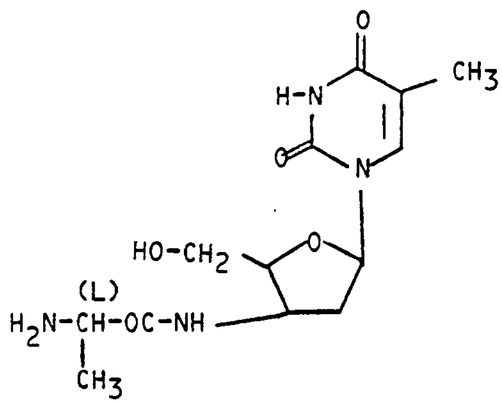

Le protocole opératoire est identique à celui décrit dans l'exemple 2.

Etape A : Synthèse de N[(N-benzyloxycarbonyl)-L-alanyl]-amino-3' didéoxy-2',3'-méthyl-5-uridine (2)

On utilise au départ 5 g (20,7 mmoles) d'amino-3'-didéoxy-2',3'-méthyl-5-uridine.

On obtient ainsi 6,14 g (13,7 mmoles) du composé (2), avec une pureté satisfaisante (Rdt : 66,5 %)
F (thermovar) : 145-148 ° C
$[\alpha]^{20}{}_{D}$ = +5 ° 9 (c = 1, MeOH)
RMN (200 MHz, $CD_3OD$) : 1,30 à 1,34 (3H, d, Me-ala) 1,68 (3H, s, C-5-4) ; 2,28 à 2,34 (2H, t, C-2'-H) 3,7 à 3,8 (3H, m, C-4'-H, C-5'-H) ; 4,07 à 4,11 (1H, m, C-) 4,47 à 4,51 (1H, m, C-3'-H), 5,07 (2H, s, $CH_2$) 6,19 à 6,22 (1H, t, C-1'-H) ; 7,27 à 7,33 (5H, m, $C_6H_5$) 7,87 (1H, s, C-6-H)

Etape B

On obtient à partir du composé (2), 3,66 g (11,7 mmoles) de N-(L-alanyl)-amino-3'-didéoxy-2',3'-méthyl-5-uridine
(Rdt : 85,9 %)
F (thermovar) : 180 ° -185 ° C
$[\alpha]_D^{20}$ = + 32 ° 4 (C = 1, MeOH)
RMN (200 Mz, $D_2O$) : 1,28 à 1,31 (3H, d, Me-ala) 1,90 (3H, s, C-5-H) ; 2,41 à 2,50 (2H, m, C-2'-H) ; 3,54 à 3,58 (1H, m, C-ala H) ; 3,76 à 4,00 (3H, m, C-4'-H C-5'-H) ; 4,49 à 4,53 (1H, m, C-3-H) ; 6,22 à 6,26 (1H, t, C-1'-H) ; 7,70 (1H, s, C-6-H)

Exemple 5 : Voie chimique

Synthèse de N-(L-alanyl)-amino-5-déoxy-2'-uridine

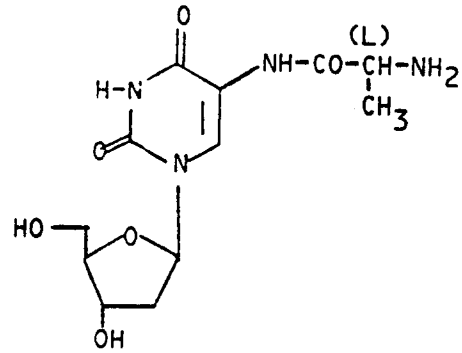

Etape A : Synthèse de N-[(N-benzyloxycarbonyl)-L-alanyl]amino-5-déoxy-2'-uridine (3)

A une solution de 6 g (21 mmoles) de chlorhydrate d'amino-5-déoxy-2'-uridine dans 220 ml de méthanol et d'eau (4 : 1) et 2,8 ml de triéthylamine sont ajoutés 10,38 g (42 mmoles) de N-(éthoxy-carbonyl)-2-éthoxy-1,2-dihydroquinoline et 9,36 g (42 mmoles) de (N-benzyloxycarbonyl)-L-alanine.

Le milieu réactionnel est conservé à température ambiante 12 heures. Après évaporation sous vide à 20 °C des solvants, l'huile obtenue est soigneusement triturée en présence d'éther de pétrole jusqu'à obtention d'une pâte. L'addition de chloroforme donne naissance à un précipité qui, après filtration, est soigneusement lavé plusieurs fois par du chloroforme et enfin par de l'éther.

Après séchage sous vide à 20 °C on obtient 8 g (18 mmoles) de N-[(N-benzyloxycabonyl)-L-alanyl] amino-5-déoxy-2'-uridine. La recristallisation dans un mélange de chloroforme et de méthanol conduit à 7,2 g (16,5 mmoles) d'un composé de pureté satisfaisante (Rdt : 77 %)
Pureté (HPLC) : 100 %
$[\alpha]_D^{20}$ = - 30 ° (C = 0,5, MeOH)
F (thermovar) = 104-107 °C
CCM Silice 60 F $_{254}$ ($CHCl_3$, EtOH : 90/10) rf = 0,176 RMN (200 MHz, $CD_3OD$) :
1,36 à 1,39 (3H, d, $CH_3$ ala) ; 2,23 à 2,26 (2H, m, C-2'-H) ; 3,72 à 3,74 (2H, d, C-5'-H) ; 3,91 à 3,93 (1H, m, C-4'-H) ; 4,31 à 4,35 (2H, m, C-3'H, CH-ala) ; 6,29 à 6,36 (1H, t, C-1'H) ; 8,61 (1H, s, C-6H)

Etape B

4 g (9 mmoles) du composé (3) sont mis en solution dans 300 ml de méthanol et hydrogénés en présence de 400 mg de charbon palladié à 10 % pendant 3 heures. Après filtration du catalyseur et concentration du solvant, on reprend le résidu par de l'éther éthylique et filtre le précipité obtenu. On obtient après séchage 2,7 g (8,6 mmoles) de N-(L-alanyl)-amino-5-déoxy-2'-uridine (Rdt : 94 %). La recristallisation dans le n-butanol conduit à 2,1 g (6,7 mmoles) du composé désiré (Rdt = 73 %)
Rdt global : 56 %
Pureté (HPLC) : 96 %
$[\alpha]_D^{20}$ = + 15 ° (C = 0,5 MeOH)
F (thermovar) : 166-168 °C
CCM silice 60$F_{254}$ (MeOH, $H_2O$ : 80/20) rf = 0,303
IR (HBr) : 1670 $cm^{-1}$ (C = O) ; 1275 $cm^{-1}$, 1580 $cm^{-1}$, 3250 cm $^{-1}$ (N-H)
RMN (200 MHz, $CD_3OD$) : 1,55 (3H, d, $CH_3$ala), 2,20 à 2,31 (2H, m, C-2'-H) ; 3,72 à 3,74 (2H, d, C-5'-H) ; 3,92 à 3,93 (1H, m, C-4'-H) ; 4,14 à 4,18 (1H, m, C-ala-H) ; 4,35 à 4,38 (1H, m, C-3'-H) ; 6,28 à 6,34 (1H, t, C-1'-H) ; 8,60 (1H, s, C-6-H).

Exemples 6 à 17

En utilisant des processus expérimentaux analogues à ceux décrits dans les exemples 1 et 2 et que l'homme de métier retrouvera facilement, on a préparé les composés suivants :

N-(L-alanyl)-amino 5'-didéoxy-2',5'-éthyl-5-uridine
N-(L-alanyl)-amino-5'-didéoxy-2',5'-butyl-5-uridine
N-(L-alanyl)-amino-5'-didéoxy-2',5'-propyl-5-uridine
N-(L-valyl)-amino-5'-didéoxy-2',5'-méthyl-5-uridine
N-(L-leucyl)-amino-5'-didéoxy-2',5'-méthyl-5-uridine
N-(sarcosyl)-amino-5'-didéoxy-2',5'-méthyl-5-uridine
N-($\beta$-alanyl)-amino-5'-didéoxy-2',5'-methyl-5-uridine
N-(L-alanyl-L-alanyl)-amino-5'-didéoxy-2',5'-methyl-5-uridine
N-(L-alanyl-L-alanyl-L-alanyl)-amino-5'-didéoxy-2',5'-méthyl-5-uridine
N-(L-alanyl)-amino-5'-didéoxy-2',5'-vinyl-5-uridine
N-(L-alanyl)-amino-5'-didéoxy-2',5'-bromovinyl-5-uridine

Exemples 18 à 31

En utilisant des processus expérimentaux analogues à ceux décrits dans l'exemple 3 et que l'homme de métier retrouvera facilement, on a préparé les composés suivants :
N-(L-alanyl)-amino-3'-didéoxy-2',3'-méthyl-5-uridine
N-(L-alanyl)-amino-3'-didéoxy-2',3'-éthyl-5-uridine
N-(L-alanyl)-amino-3'-didéoxy-2',3'-butyl-5-uridine
N-(L-alanyl)-amino-3'-didéoxy-2',3'-propyl-5-uridine
N-(L-valyl)-amino-3'-didéoxy-2',3'-méthyl-5-uridine
N-(L-leucyl)-amino-3'-didéoxy-2',3'-méthyl-5-uridine
N-(sarcosyl)-amino-3'-didéoxy-2',3'-méthyl-5-uridine
N-($\beta$-alanyl)-amino-3'-didéoxy-2',3'-méthyl-5-uridine
N-(L-alanyl-L-alanyl)-amino-3'-didéoxy-2',3'-méthyl-5-uridine
N-(L-alanyl-L-alanyl-L-alanyl)-amino-3'-didéoxy-2',3'-méthyl-5-uridine
N-(L-glycyl)-amino-3'-didéoxy-2',3'-méthyl-5-uridine
N-(L-alanyl)$_6$-amino-3'-didéoxy-2',3'-méthyl-5-uridine
N-(L-alanyl)-amino-3'-didéoxy-2',3'-vinyl-5-uridine
N-(L-alanyl)-amino-3'-didéoxy-2,3'-bromovinyl-5-uridine

Exemples 32 à 38

En utilisant des processus expérimentaux analogues à celui décrit dans l'exemple 5 et que l'homme de métier retrouvera facilement, on a préparé les composés suivants :
N-(L-valyl)-amino-5 déoxy-2'-uridine
N-(L-leucyl)-amino-5 déoxy-2'-uridine
N-(sarcosyl)-amino-5-déoxy-2'-uridine
N-($\beta$-alanyl)-amino-5-déoxy-2'-uridine
N-(L-alanyl-L-alanyl)-amino-5-déoxy-2'-uridine
N-(L-alanyl)$_6$-amino-5-déoxy-2'-uridine
N-(L-glycyl-L-alanyl)-amino-5-déoxy-2'-uridine

L'activité des composés selon l'invention, comme inhibiteurs de la synthèse de l'ADN des cellules cancéreuses a été évaluée :

- "in vitro" sur la TKF cytosolique (étude 1)
- "in vivo" sur les cellules MCF7 en culture (étude 2)
- "in vivo" sur des rats femelles WISTAR immatures (étude 3)

Etude 1

Des cellules du cancer du sein hormonodépendantes (MCF7) qui contiennent 95-96 % de thymidine kinase foetale, sont traitées par les ultrasons et centrifugées à 105.000 g.

Les cellules MCF7 proviennent d'une métastase pleurale d'un cancer du sein humain (Ref. H. SOULE, J. VASQUEZ, A. LONG, S. ALBERT and M. BREUNAN. J. Natl. Cancer Instit., 51, 1409-1416, 1973) et sont par exemple disponibles à l'A.T.C.C. (American Type Culture Collection).

La concentration des protéines du cytosol obtenu est mesurée et ajustée par dilution à 1,5 mg de protéines par ml de cytosol.

Celui-ci est incubé, à 37°C pendant 10 minutes, dans un tampon Tris (pH : 7,6) en présence de thymidine [$C^{14}$-2] (40 µmolaire), d'ATP (8 mMolaire) et de chlorure de magnésium. La réaction enzymatique est stoppée par l'addition d'acide perchlorique.

Les nucléotides formés sont séparés par électrophorèse haute tension, les taches sont découpées et comptées.

On a donné au Tableau I les résultats des essais réalisés avec les composés des exemples 1 et 2 ainsi qu'avec des témoins sans inhibiteur, à titre de comparaison.

Les valeurs données sont exprimées en picomoles par millilitre de cytosol par minute.

TABLEAU 1

| Composé | Concentration | TMP + TTP | TTP |
|---|---|---|---|
| (Témoin)<br>Ex. 1 | sans inhibiteur<br>1 mMolaire | 1993<br>1329 | 1573<br>38 |
| (Témoin)<br>Ex. 2 | sans inhibiteur<br>1 mMolaire | 486<br>388 | 368<br>46 |

Ce test biochimique est basé sur la connaissance du fait que la thymidine kinase occupe une position stratégique dans les processus biochimiques aboutissant à la synthèse de l'ADN, qui utilise le thymidine triphosphate (TTP) qui peut être considéré comme le produit final d'une réaction complexe initiée par la thymidine kinase.

Comme le montre le tableau 1, les composés selon l'invention agissent comme inhibiteurs de la synthèse du TTP, et donc vraisemblablement de la synthèse de l'ADN des cellules cancéreuses en voie de prolifération.

Etude 2

Culture des cellules MCF-7

Des cellules MCF-7 sont cultivées dans des flacons de 75 $cm^2$. Chaque flacon est ensemencé avec 0,6 x $10^6$ cellules en suspension dans 10 ml de milieu RPMI-1640 auquel on ajoute 2 mM de L-glutamine, 2,5 µg/ml de Fungizone, 15 U/ml de Pénicilline, 50 µg/ml de Streptomycine et 5 % de sérum de veau foetal.

Les cellules sont cultivées pendant 72 heures à 37°C dans un incubateur à $CO_2$ (EG 110, IR. Jouan). Les milieux de culture sont changés toutes les 48 heures.

La récolte des cellules est effectuée par décollement au moyen d'un rubber-policeman et la suspension de cellules est centrifugée à 200 g dans une centrifugeuse réfrigérée pour obtenir un culot de cellules.

Essais des inhibiteurs

Les différents inhibiteurs à tester sont dissous dans du RPMI-1640 à la concentration de 1 mM. Leurs effets sont testés pendant 48 et 72 heures.

Tests analytiques

- Comptage des cellules : Les tapis cellulaires sont lavés, 3 fois, avec 5 ml d'un tampon phosphate salin (PBS). Le contenu de chaque flacon de culture est, ensuite, mis en suspension dans 10 ml de tampon PBS. Les cellules sont comptées au moyen d'un compteur Hycel Counter H.C. 202 [R].
- Dosage de l'ADN : Il est effectué sur une partie aliquote du sonicat de cellules et par spectrofluorimétrie selon la technique de C. Brunk et al., Analyst. Biochem., 92, 497-500 1979.

TABLEAU 2

| | 48 h | | 72 h | |
|---|---|---|---|---|
| | Cellules x 10 $^6$/boîte | ADN $\mu$g/boîte | Cellules x 10 $^6$/boîte | ADN $\mu$g/boîte |
| Témoins | 2,41 | 47,4 | 5,03 | 89,8 |
| Exemple 1 | 0,60 | 8,1 | 0,43 | 2,5 |
| Exemple 2 | 1,50 | 22,7 | 3,60 | 56,5 |
| Exemple 4 | 1,20 | 16,2 | 2,55 | 34,8 |

Etude 3

PROTOCOLE

Animaux — Rats femelles WISTAR (IFFA CREDO) âgés de 22 jours de poids moyen compris entre 30 et 50 g.
Ils sont séparés de la mère 2 jours avant le début de l'essai.
Ils ont libre accès à la nourriture et à l'eau de boisson pendant la durée de l'essai.

Produits

- NaCl à 0,9 %
- Alcool absolu
- Dipropionate de 17 $\beta$ oestradiol (Sigma référence E.9125)
  Une solution à 2 mg.ml$^{-1}$ dans NaCl à 0,9 % est effectuée
- 6 -[$^3$H] Déoxythymidine (Amersham référence TRK61 à 24 Ci/mmole
  La solution de travail est préparée par dilution de la solution commerciale au 1/11ème.
  Son activité volumique est de 10 $\mu$Ci/0,1 ml
- Exemple 1
- Exemple 2
  Ces produits sont mis en solution dans NaCl 0,9 % de façon à administrer un volume de 0,1 ml/animal
- NaOH 5 ml
- $HClO_4$ 0,6 N
- KOH 0,6 N
- Diphénylamine (Sigma référence D.2285) en solution dans l'acide acétique glacial (1 %)
- $H_2SO_4$ concentré
- ADN de Thymus de veau (Borhinger référence 104 175) en solution dans NaOH 5 mM
- Instagel ®

Mode Opératoire

1. Traitements et sacrifice

Ils sont effectués selon le schéma suivant :

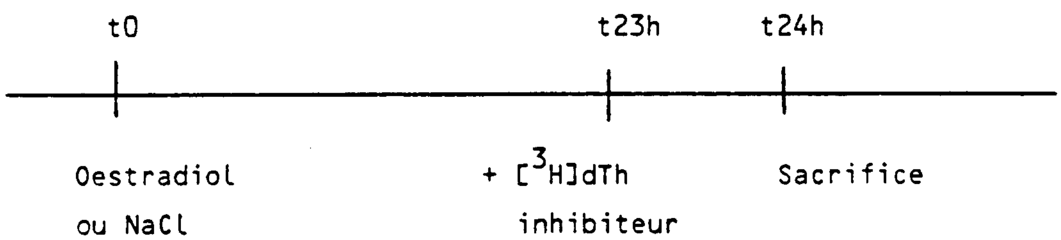


t0 — Le lot "Témoin Absolu" reçoit 0,1 ml de NaCl à 0,9 % par voie intrapéritonéale. Les autres

lots reçoivent 800 ng/animal d'oestradiol sous un volume de 0,1 ml.

t23h Tous les animaux reçoivent 10 µCi de [$^3$H]dTh sous un volume de 0,1 ml par voie intrapéritonéale.

Le lot "Témoin Oestradiol" et le lot "Témoin Absolu" reçoivent 0,1 ml de NaCl à 0,9 %.

Les lots "Inhibiteurs" reçoivent les produits à tester sous un volume de 0,1 ml par voie intrapéritonéale.

t24h Tous les animaux sont sacrifiés par dislocation cervicale. Les utérus sont prélevés, essuyés sur papier filtre, pesés et congelés.

2. Traitement des utérus

L'utérus est broyé dans 0,5 ml d'Acide Perchlorique 0,6 N à l'aide du Potter. Le piston du broyeur est rincé à 3 reprises avec 0,3 ml d'Acide Perchlorique 0,6 N. L'homogénat et les liquides de rinçage sont rassemblés dans des tubes coniques en verre, puis centrifugés à 800 g pendant 10 minutes. Le culot est lavé 2 fois avec 1 ml d'Acide Perchlorique 0,3 N.

Ce culot est repris par 0,6 ml de KOH 0,3 N ; les tubes sont placés au bain-marie à 37 °C pendant 2 heures 30.

Après hydrolyse alcaline (Hydrolyse de l'ARN) 30 µl d'Acide Perchlorique 11 N sont ajoutés afin de précipiter l'ADN.

Les tubes sont refroidis dans la glace puis centrifugés à 4200 g pendant 10 minutes.

Le culot est lavé 2 fois avec 1 ml d'Acide Perchlorique 0,3 N. Le dernier culot est repris par 0,6 ml d'Acide Perchlorique 0,6 N. Les tubes sont placés 10 minutes à 80 °C. Après refroidissement dans la glace pendant quelques minutes ils sont centrifugés pendant 10 minutes à 4200 g.

3. Détermination de la radioactivité incorporée dans l'ADN

50 µl de surnageant sont placés dans des fioles de comptage. Après addition de 5 ml d'Instagel, le comptage est effectué sur 10 minutes.

Les c.p.m. (coups par minute) sont convertis en d.p.m. :

$$d.p.m. = \frac{c.p.m.}{E} \quad (d.p.m. = \text{désintégrations par minute})$$

E = efficacité de comptage = 0,37

Les d.p.m. en picomoles de [$^3$H] dTh selon la formule

$$\frac{d.p.m.}{2220 \times 25}$$

Activité spécifique de la [$^3$H] dTh : 25 nCi/picomole 1nCi = 2220 d.p.m.

Les résultats sont exprimés en pmole de Déoxythymidine incorporée par utérus.

4. Dosage de l'A.D.N.

A 100 µl de surnageant, dilué au 1/6 dans l'Acide Perchlorique 0,6 N sont ajoutés 1,2 ml d'une solution de diphénylamine dans l'Acide Acétique glacial (1 g/100 ml).

Les tubes sont placés 10 minutes au bain-marie bouillant. Après refroidissement, la Densité Optique est lue à 595 nm. L'étalonnage est effectué à l'aide d'une solution d'ADN de Thymus de Veau.

Les résultats sont exprimés en µg/utérus.

TABLEAU 3

| | Poids des utérus mg | A.D.N. µg/utérus | 3H - dTh pH/utérus |
|---|---|---|---|
| Témoin (n = 8) | 33,1 ± 1,2 | 273 ± 18 | 0,189 ± 0,004 |
| Oestradiol (n = 8) | 44,4 ± 5,4 | 371 ± 49 | 1,133 ± 0,244 |
| Oestradiol + Exemple 1 10 $mg.kg^{-1}$ (n = 8) | 55,2 ± 4,9 | 383 ± 32 | 0,735 ± 0,152 |
| Oestradiol + Exemple 2 10 $mg.kg^{-1}$ (n = 8) | 54,5 ± 3,9 | 360 ± 17 | 0,708 ± 0,165 |

## Revendications

**1.** Dérivés de la déoxy-2'-uridine, caractérisés en ce qu'ils répondent à la formule générale suivante :

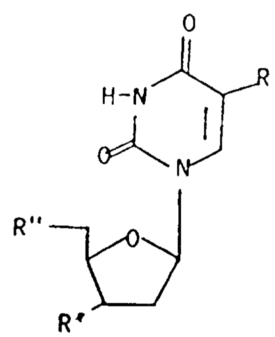

(I)

dans laquelle

- . R est choisi parmi un radical alkyle ou alcényle ayant de 1 à 4 atomes de carbone, un radical aryle ou un halogène, lesdits radicaux alkyle, alcényle et aryle pouvant comporter au moins un substituant halogène ; et un radical de formule $-NH-R_1$, dans lequel $R_1$ représente un reste d'acide aminé ou de peptide comportant de 2 à 6 acides aminés ;
- . R' et R'' étant choisis parmi un radical hydroxy et un radical de formule $-NH-R_1$, dans lequel $R_1$ a la même signification que ci-dessus ;

à la condition que l'un au moins de R, R' et R'' représente un radical de formule $-NH-R_1$ ; que R' et R'' ne soient pas simultanément $-NH-R_1$, et que lorsque R est $-NH-R_1$, R' et R'' soient simultanément un groupe hydroxy et $R_1$ ne représente pas un reste d'acide aminé $\omega$ ; et leurs sels acceptables en pharmacie.

**2.** Dérivés selon la revendication 1, de formule générale (I), dans laquelle R' est un groupe hydroxy et R'' est un groupe $-NH-R_1$.

**3.** Dérivés selon la revendication 1, de formule générale (I), dans laquelle R'' est un groupe hydroxy et R' est un groupe $-NH-R_1$.

**4.** Dérivés selon la revendication 1, de formule générale (I), dans laquelle R est un groupe $-NH-R_1$.

**5.** Dérivés selon l'une des revendications 1 à 4, caractérisés en ce que le reste d'acide aminé ou de peptide précité se présente sous la forme L ou D.

**6.** Dérivés selon la revendication 1, caractérisés en ce qu'il s'agit des composés suivants :

- . N-(L-alanyl)-amino-5'-didéoxy-2',5'-méthyl-5-uridine
- . N-(D-alanyl)-amino-5'-didéoxy-2',5'-méthyl-5-uridine
- . N-(D-alanyl)-amino-3'-didéoxy-2',3'-méthyl-5-uridine
- . N-(L-alanyl)-amino-3'-didéoxy-2',3'-méthyl-5-uridine
- . N-(L-alanyl)-amino-5-déoxy-2'-uridine.

**7.** Procédé de préparation des dérivés répondant à la formule générale (I) selon la revendication 1, caractérisé en ce qu'il comporte la réaction, par voie enzymatique ou par voie chimique, d'un dérivé aminé répondant à la formule générale :

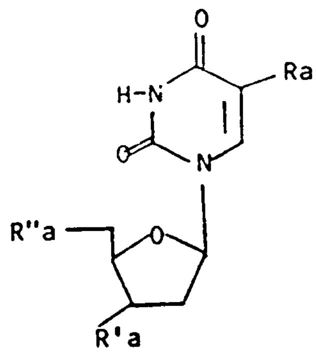

(Ia)

dans laquelle

$R_a$ est choisi parmi un radical alkyle ou alcényle ayant de 1 à 4 atomes de carbone, un radical aryle ou un halogène, lesdits radicaux alkyle, alcényle et aryle pouvant comporter au moins un substituant halogène, et un radical $-NH_2$ ;

$R'_a$ et $R''_a$ étant choisis parmi un radical hydroxy et un radical $-NH_2$,

à la condition que $R'_a$ et $R''_a$ ne soient pas simultanément $-NH_2$ et que, lorsque $R_a$ est $-NH_2$, $R'_a$ et $R''_a$ soient simultanément un groupe hydroxy ;

avec un acide aminé ou un peptide comportant de 2 à 6 acides aminés, étant entendu que lorsque $R_a$ est $-NH_2$ cet acide aminé ne soit pas un acide aminé $\omega$ et, si on le désire, la conversion d'un composé ainsi obtenu en un sel acceptable en pharmacie.

**8.** Procédé de préparation des dérivés répondant à la formule générale (I) selon la revendication 1, dans laquelle le reste d'acide aminé ou de peptide se présente sous la forme L, caractérisé en ce qu'il comporte la réaction enzymatique des dérivés de formule générale (Ia) telle que définie à la revendication 7 avec un acide aminé ou un peptide comportant de 2 à 6 acides aminés, en présence d'une enzyme choisie parmi la papaïne et la chymopapaïne, la réaction étant effectuée à une température d'environ 10 à environ 70 °C en milieu acide, les fonctions amines dudit acide aminé ou dudit peptide ayant été préalablement bloquées.

**9.** Composition pharmaceutique, caractérisée en ce qu'elle contient, à titre d'ingrédient actif, au moins un composé répondant à la formule générale suivante :

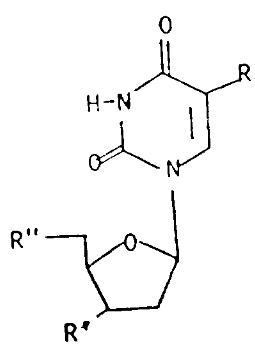

(I)

dans laquelle

- . R est choisi parmi un radical alkyle ou alcényle ayant de 1 à 4 atomes de carbone, un radical aryle ou un halogène, lesdits radicaux alkyle, alcényle et aryle pouvant comporter au moins un substituant halogène ; et un radical de formule $-NH-R_1$, dans lequel $R_1$ représente un reste d'acide aminé ou de peptide comportant de 2 à 6 acides aminés ;

. R' et R'' étant choisis parmi un radical hydroxy et un radical de formule -NH-$R_1$, dans lequel $R_1$ a la même signification que ci-dessus ;
à la condition que l'un au moins de R, R' et R'' représente un radical de formule -NH-$R_1$ ; que R' et R'' ne soient pas simultanément -NH-$R_1$, et que lorsque R est -NH-$R_1$, R' et R'' soient simultanément un groupe hydroxy ; et leurs sels acceptables en pharmacie, en association avec un véhicule ou support non toxique, pharmaceutiquement acceptable.

**10.** Composition pharmaceutique à activité d'inhibiteur de la synthèse de l'ADN des cellules cancéreuses en voie de prolifération, caractérisée en ce qu'elle contient, à titre d'ingrédient actif, au moins un composé répondant à la formule générale suivante :

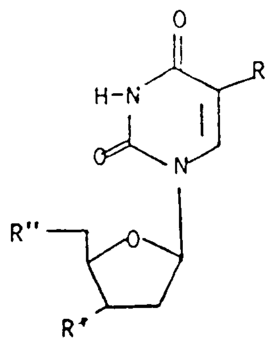

(I)

dans laquelle
. R est choisi parmi un radical alkyle ou alcényle ayant de 1 à 4 atomes de carbone, un radical aryle ou un halogène, lesdits radicaux alkyle, alcényle et aryle pouvant comporter au moins un substituant halogène ; et un radical de formule -NH-$R_1$, dans lequel $R_1$ représente un reste d'acide aminé ou de peptide comportant de 2 à 6 acides aminés ;
. R' et R'' étant choisis parmi un radical hydroxy et un radical de formule -NH-$R_1$, dans lequel $R_1$ a la même signification que ci-dessus ;
à la condition que l'un au moins de R, R' et R'' représente un radical de formule NH-$R_1$ ; que R' et R'' ne soient pas simultanément -NH-$R_1$, et que lorsque R est -NH-$R_1$, R' et R'' soient simultanément un groupe hydroxy ; et leurs sels acceptables en pharmacie, en association avec un véhicule ou support non toxique, pharmaceutiquement acceptable.

## Claims

**1.** 2'-deoxyuridine derivatives, characterized in that they have the following general formula:

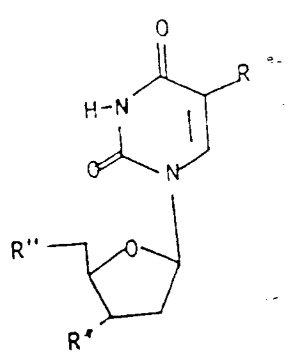

(I)

in which
- R is selected from an alkyl or alkenyl radical having from 1 to 4 carbon atoms, an aryl radical or a halogen, it being possible for said alkyl, alkenyl and aryl radicals to contain at least one halogen substituent; and a radical of the formula -NH-$R_1$, in which $R_1$ is an amino acid residue or a peptide residue containing from 2 to 6 amino acids;

- R' and R" being selected from a hydroxyl radical and a radical of the formula $-NH-R_1$, in which $R_1$ is as defined above;

with the proviso that at least one of R, R' and R" represents a radical of formula $-NH-R_1$; that R' and R" are not simultaneously $-NH-R_1$ and that, when R is $-NH-R_1$, R' and R" are simultaneously a hydroxy group and $R_1$ does not represent a ω-amino acid residue; and their pharmaceutically acceptable salts.

**2.** Derivatives according to claim 1 of general formula (I) in which R' is a hydroxyl group and R" is a group $-NH-R_1$.

**3.** Derivatives according to claim 1 of general formula (I) in which R" is a hydroxyl group and R' is a group $-NH-R_1$.

**4.** Derivatives according to claim 1 of general formula (I) in which R is a group $-NH-R_1$.

**5.** Derivatives according to one of claims 1 to 4, characterized in that the amino acid or peptide residue mentioned above is in the L or D form.

**6.** Derivatives according to claim 1, characterized in that it is one of the following compounds:
   * 5'-N-(L-alanyl)amino-2', 5'-dideoxy-5-methyluridine
   * 5'-N-(D-alanyl)amino-2', 5'-dideoxy-5-methyluridine
   * 3'-N-(D-alanyl)amino-2', 3'-dideoxy-5-methyluridine
   * 3'-N-(L-alanyl)amino-2', 3'-dideoxy-5-methyluridine
   * 5-N-(L-alanyl)amino-2'-deoxyuridine.

**7.** Process for the preparation of the derivatives of general formula (I) according to claim 1, characterized in that it comprises reacting an amine derivative of the general formula:

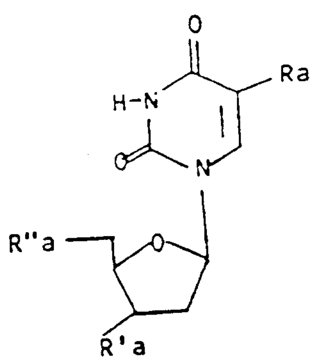

(Ia)

in which

Ra is selected from an alkyl or alkenyl radical having from 1 to 4 carbon atoms, an aryl radical or a halogen, it being possible for said alkyl, alkenyl and aryl radicals to contain at least one halogen substituent, and a radical $-NH_2$;

R'a and R"a being selected from a hydroxyl radical and a radical-$NH_2$,with the proviso that R'a and R"a are not simultaneously $-NH_2$ and that, when Ra is $-NH_2$, R'a and R"a are simultaneously a hydroxyl group,

with an amino acid or a peptide containing from 2 to 6 amino acids, by an enzymatic or chemical method, it being understood that when Ra is $-NH_2$ this amino acid is not a ω-amino acid and, if desired, converting a compound obtained in this way into a pharmaceutically acceptable salt.

**8.** Process for the preparation of the derivatives of general formula (I) according to claim 1, in which the amino acid or peptide residue is in the L form, characterized in that it consists of the enzymatic reaction of the derivatives of general formula (Ia), as defined in claim 7, with an amino acid or a peptide containing from 2 to 6 amino acids, in the presence of an enzyme selected from papain and chymopapain, the reaction being carried out at a temperature of about 10 to about 70°C in an acid medium, the amine groups of said amino acid or of said peptide having been blocked beforehand.

**9.** Pharmaceutical composition, characterized in that it contains, as the active ingredient, at least one compound of the following general formula:

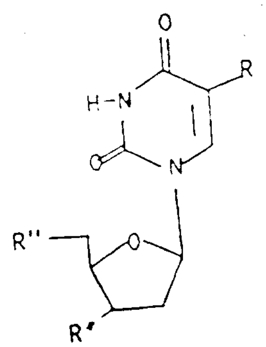

(I)

in which

- R is selected from an alkyl or alkenyl radical having from 1 to 4 carbon atoms, an aryl radical or a halogen, it being possible for said alkyl, alkenyl and aryl radicals to contain at least one halogen substituent; and a radical of the formula -NH-$R_1$, in which $R_1$ is an amino acid residue or a peptide residue containing from 2 to 6 amino acids;
- R' and R'' being selected from a hydroxyl radical and a radical of the formula -NH-$R_1$, in which $R_1$ is as defined above;

with the proviso that at least one of R, R' and R'' represents a radical of formula -NH-$R_1$; that R' and R'' are not simultaneously -NH-$R_1$ and that, when R is -NH-$R_1$, R' and R'' are simultaneously a hydroxy group; and their pharmaceutically acceptable salts, in association with a pharmaceutically acceptable, non-toxic vehicle or carrier.

**10.** Pharmaceutical composition active as an inhibitor of DNA synthesis in proliferating cancerous cells, characterized in that it contains, as the active ingredient, at least one compound of the following general formula:

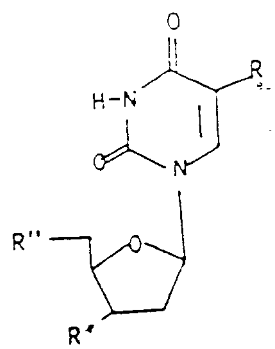

(I)

in which

- R is selected from an alkyl or alkenyl radical having from 1 to 4 carbon atoms, an aryl radical or a halogen, it being possible for said alkyl, alkenyl and aryl radicals to contain at least one halogen substituent; and a radical of the formula -NH-$R_1$, in which $R_1$ is an amino acid residue or a peptide residue containing from 2 to 6 amino acids;
- R' and R'' being selected from a hydroxyl radical and a radical of the formula -NH-$R_1$, in which $R_1$ is as defined above,

with the proviso that at least one of R, R' and R'' represents a radical of formula -NH-$R_1$; that R' and R'' are not simultaneously -NH-$R_1$ and that, when R is -NH-$R_1$, R' and R'' are simultaneously a hydroxy group; and their pharmaceutically acceptable salts, in association with a pharmaceutically acceptable, non-toxic vehicle or carrier.

## Patentansprüche

1. Derivate von 2-Desoxyuridin, gekennzeichnet durch die folgende allgemeine Formel

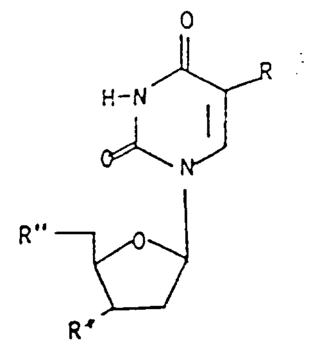

(I),

in der bedeuten:
- R $C_{1-4}$-Alkyl, $C_{1-4}$-Alkenyl oder Aryl, wobei die Alkyl-, Alkenyl- und Arylgruppen mindestens einen Halogensubstituenten enthalten können, Halogen oder eine Gruppe der Formel -NH-$R_1$, in der $R_1$ einen Aminosäurerest oder einen Peptidrest darstellt, der 2 bis 6 Aminosäuren enthält, und
- R' und R" Hydroxy oder eine Gruppe der Formel -NH-$R_1$ mit $R_1$ wie oben,

mit den Maßgaben, daß mindestens eine der Gruppen R, R' und R" eine Gruppe der Formel -NH-$R_1$ darstellt, daß R' und R" nicht gleichzeitig -NH-$R_1$ sind, sowie, daß, wenn R -NH-$R_1$ ist, R' und R" gleichzeitig Hydroxy bedeuten und $R_1$ kein Rest einer $\omega$-Aminosäure ist,
sowie ihre pharmazeutisch akzeptablen Salze.

2. Derivate der allgemeinen Formel (I) nach Anspruch 1, wobei $R_1$ Hydroxy und $R_2$ eine Gruppe -NH-$R_1$ darstellen.

3. Derivate der allgemeinen Formel (I) nach Anspruch 1, wobei R" Hydroxy und R' eine Gruppe -NH-$R_1$ darstellen.

4. Derivate der allgemeinen Formel (I) nach Anspruch 1, wobei R eine Gruppe -NH-$R_1$ darstellt.

5. Derivate nach einem der Ansprüche 1 bis 4, dadurch gekennzeichnet, daß der vorgenannte Aminosäure- bzw. Peptidrest in L- oder D-Form vorliegt.

6. Derivate nach Anspruch 1, dadurch gekennzeichnet, daß es sich um folgende Verbindungen handelt:
   - 5'-N-(L-Alanyl)-amino-2',5'-didesoxy-5-methyluridin
   - 5'-N-(D-Alanyl)-amino-2',5'-didesoxy-5-methyluridin
   - 3'-N-(D-Alanyl)-amino-2',3'-didesoxy-5-methyluridin
   - 3'-N-(L-Alanyl)-amino-2',3'-didesoxy-5-methyluridin
   - 5-N-(L-Alanyl)-amino-2'-desoxyuridin.

7. Verfahren zur Herstellung der Derivate der allgemeinen Formel (I) nach Anspruch 1, gekennzeichnet durch enzymatische oder chemische Umsetzung eines Aminoderivats der allgemeinen Formel

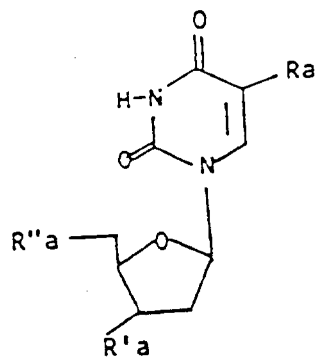

(Ia) '

in der bedeuten:

- $R_a$ $C_{1-4}$-Alkyl, $C_{1-4}$-Alkenyl oder Aryl, wobei die Alkyl-, Alkenyl- und Arylgruppen mindestens einen Halogensubstituenten enthalten können, Halogen oder eine Gruppe $-NH_2$ und
- R' und R'' Hydroxy oder $-NH_2$,

mit den Maßgaben, daß $R'_a$ und $R''_a$ nicht gleichzeitig $-NH_2$ sind, sowie, daß, wenn $R_a$ $-NH_2$ ist, $R'_a$ und $R''_a$ gleichzeitig Hydroxy bedeuten,
mit einer Aminosäure oder einem aus 2 bis 6 Aminosäuren bestehenden Peptid, wobei gilt, daß diese Aminosäure keine ω-Aminosäure ist, wenn $R_a$ $-NH_2$ ist,
sowie gegebenenfalls Umwandlung der nach dem obigen Verfahren erhaltenen Verbindung in ein pharmazeutisch akzeptables Salz.

**8.** Verfahren zur Herstellung der Derivate der allgemeinen Formel (I) nach Anspruch 1, in welcher der Aminosäure- oder Peptidrest in der L-Form vorliegt, gekennzeichnet durch enzymatische Umsetzung von Derivaten der in Anspruch 7 angegebenen allgemeinen Formel (Ia) mit einer Aminosäure oder einem aus 2 bis 6 Aminosäuren bestehenden Peptid in Gegenwart eines unter Papain und Chymopapain ausgewählten Enzyms, wobei die Umsetzung bei einer Temperatur von etwa 10 bis etwa 70 °C in saurem Medium durchgeführt wird und die Aminofunktionen der oben erwähnten Aminosäure oder des erwähnten Peptids zuvor blockiert wurden.

**9.** Pharmazeutische Zusammensetzung, dadurch gekennzeichnet, daß sie als Wirkstoff mindestens eine Verbindung der folgenden allgemeinen Formel

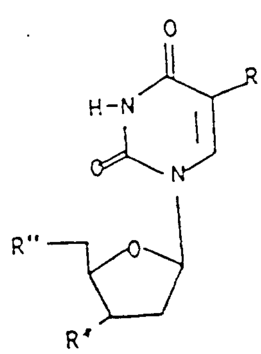

(I)

enthält, in der bedeuten:

- R $C_{1-4}$-Alkyl, $C_{1-4}$-Alkenyl oder Aryl, wobei die Alkyl-, Alkenyl- und Arylgruppen mindestens einen Halogensubstituenten enthalten können, Halogen oder eine Gruppe $-NH-R_1$, in der $R_1$ einen Aminosäurerest oder einen Peptidrest darstellt, der 2 bis 6 Aminosäuren enthält,
  und
- R' und R'' Hydroxy oder eine Gruppe der Formel $-NH-R_1$ mit $R_1$ wie oben,

mit den Maßgaben, daß mindestens eine der Gruppen R, R' und R'' eine Gruppe der Formel $-NH-R_1$ darstellt, daß R' und R'' nicht gleichzeitig $-NH-R_1$ sind, sowie, daß, wenn R $-NH-R_1$ ist, R' und R''

gleichzeitig Hydroxy bedeuten,
und ihre pharmazeutisch akzeptablen Salze in Kombination mit einem pharmazeutisch akzeptablen, nichttoxischen Vehikel oder Träger.

**10.** Pharmazeutische Zusammensetzung mit hemmender Wirkung auf die DNA-Synthese von Krebszellen bei der Proliferation, dadurch gekennzeichnet, daß sie als Wirkstoff mindestens eine Verbindung der folgenden allgemeinen Formel

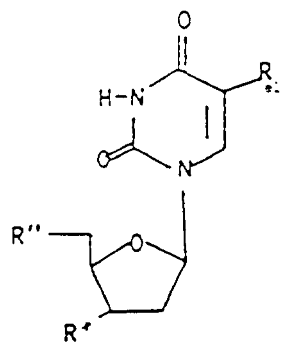

(I)

enthält, in der bedeuten:

- R $C_{1-4}$-Alkyl, $C_{1-4}$-Alkenyl oder Aryl, wobei die Alkyl-, Alkenyl- bzw. Arylgruppen mindestens einen Halogensubstituenten enthalten können, Halogen oder eine Gruppe der Formel $-NH-R_1$, in der $R_1$ einen Aminosäurerest oder einen Peptidrest darstellt, der 2 bis 6 Aminosäuren enthält, und
- R' und R'' Hydroxy oder eine Gruppe der Formel $-NH-R_1$ mit $R_1$ wie oben,

mit den Maßgaben, daß mindestens eine der Gruppen R, R' und R'' eine Gruppe der Formel $-NH-R_1$ darstellt, daß R' und R'' nicht gleichzeitig $-NH-R_1$ sind, sowie, daß, wenn R $-NH-R_1$ ist, R' und R'' gleichzeitig Hydroxy bedeuten,
und ihre pharmazeutisch akzeptablen Salze in Kombination mit einem pharmazeutisch akzeptablen, nichttoxischen Vehikel oder Träger.